# FASCICULE DE BREVET EUROPEEN

(11) **EP 0 617 599 B1**
(45) Date de publication et mention de la délivrance du brevet: **16.10.1996**
(21) Numéro de dépôt: 93902353.7
(22) Date de dépôt: 21.12.1992
(51) Int. Cl.: A61F 7/00, A61B 17/22

(54) **APPAREIL DE THERAPIE PAR ULTRASONS EMETTANT DES ONDES ULTRASONIQUES PRODUISANT DES EFFETS THERMIQUES ET DES EFFETS DE CAVITATION**
SCHALLWELLEN AUSSENDENDE,THERMISCHE EFFEKTE UND KAVITATIONSEFFEKTE ERZEUGENDE VORRICHTUNG FUR DIE ULTRASCHALLTHERAPIE
ULTRASONIC THERAPY APPARATUS DELIVERING ULTRASONIC WAVES WITH THERMAL AND CAVITATIONAL EFFECTS

(30) Priorité: 20.12.1991 FR 9115942; 20.12.1991 FR 9115943
(43) Date de publication de la demande: 05.10.1994
(73) Titulaire: TECHNOMED MEDICAL SYSTEMS, 69500 Bron (FR); INSTITUT NATIONAL DE LA SANTE ET DE LA RECHERCHE MEDICALE (INSERM), 75654 Paris Cédex 13 (FR)
(72) Inventeur: CHAPELON, Jean-Yves, F-69100 Villeurbanne (FR); CATHIGNOL, Dominique, F-69740 Genas (FR); GELET, Albert, F-69006 Lyon (FR); BLANC, Emmanuel, F-69230 S.-Genis-Laval (FR)
(74) Mandataire: Hirsch, Marc-Roger
(86) Numéro de dépôt international: FR9201210
(87) Numéro de publication internationale: WO9312742

(56) Documents cités:
- EP-A- 0 330 816
- EP-A- 0 363 239
- GB-A- 820 814
- GB-A- 2 167 305
- US-A- 2 559 227

## Description

La présente invention concerne essentiellement un appareil de thérapie par ultrasons émettant des ondes ultrasoniques produisant des effets thermiques et des effets de cavitation.

La présente invention concerne encore un appareil de thérapie par ultrasons équipé d'un dispositif de refroidissement.

Elle concerne enfin un procédé de commande d'un appareil de thérapie ultrasonore. Un appareil de thérapie par ultrasons comprenant les caractéristiques du préambule de la revendication 1 est connu de EP-A-0 363 239. Un autre appareil est connu de GB-A-2 167 305.

Il est connu qu'un champ acoustique ultrasonore focalisé de forte puissance peut détruire des tissus du corps humain (voir document FRY WO-89/07907 et WO-89/07909).

Il a également été décrit par DUNN et FRY dans "ultrasonic threshold dosage for the mamalian central nervous system" IEEE transactions volume BME 18, pages 253-256 que ce processus de destruction fait intervenir deux effets, à savoir un effet thermique et un effet de cavitation.

L'effet thermique est prépondérant lorsque l'intensité acoustique au point de focalisation reste en deçà d'un seuil déterminé d'environ 150 W/cm² à 1 MHz. Cet effet thermique est dû à l'absorption acoustique des tissus qui convertit l'énergie mécanique de l'onde acoustique en énergie thermique.

L'effet de cavitation devient prépondérant lorsque l'intensité acoustique au point de focalisation dépasse un seuil de 150 W/cm². Cet effet de cavitation est lié à la formation de microbulles de gaz qui explosent lorsqu'elles atteignent un diamètre critique en libérant localement une énergie considérable qui provoque la destruction des tissus voisins.

Pour obtenir une destruction tissulaire par effet thermique exclusif, il faut que le champ acoustique permette d'atteindre un seuil de destruction appelé "dose thermique". Ce seuil est fonction de la température atteinte et de la durée d'application. Il est ainsi possible de détruire des tissus en appliquant une élévation de température modérée pendant une durée d'application longue ou, au contraire, en appliquant une élévation de température importante pendant une durée d'application courte.

L'élévation de température est directement liée à l'intensité acoustique du champ ultrasonore au point focal.

Dans le cas d'une température modérée et d'une durée longue d'application, il se produit un phénomène de diffusion thermique autour du point focal, notamment par conduction de la chaleur par le milieu et par le flux sanguin, qui conduit à une mauvaise maîtrise du volume de traitement pouvant entraîner une destruction des zones saines et par conséquent nuire à la qualité du traitement.

Dans le cas d'une température élevée et d'une durée d'application brève, l'intensité acoustique au point focal dépasse le seuil de cavitation précité d'où l'obtention d'effets de cavitation qui ont un important pouvoir destructeur. Cet effet de cavitation est particulièrement important aux différentes interfaces rencontrées par le champ acoustique, par exemple la peau, les muscles et les parois des organes. On aboutit alors à une destruction tissulaire mal maîtrisée car non limitée à la zone focale du transducteur.

La présente invention a donc pour but principal de résoudre le nouveau problème technique consistant en la fourniture d'une solution permettant de créer une lésion tissulaire strictement limitée au point focal du dispositif de traitement comprenant au moins un élément transducteur piézoélectrique, et limitant ou évitant les phénomènes de diffusion thermique autour du point focal et limitant exclusivement les phénomènes de cavitation au point focal ou à la zone focale, sans production de phénomène de cavitation sensible en dehors du point ou de la zone focale.

La présente invention a encore pour but de résoudre le nouveau problème technique consistant en la fourniture d'une solution permettant de réaliser une lésion tissulaire strictement limitée au point focal du dispositif de traitement comprenant au moins un élément transducteur piézoélectrique tout en permettant de réaliser un traitement point par point de l'ensemble de la zone tissulaire de la cible à traiter, par exemple des tumeurs bénignes et malignes bien connues à l'homme de l'art, quelles soient externes ou internes. Des applications actuellement préférées sont le traitement des tumeurs bénignes et malignes du foie, de la prostate, du rein, du sein, de la peau, du cerveau, et le traitement des varicosités ainsi que de l'oesophage.

Encore un autre but principal de la présente invention est de résoudre le nouveau problème technique consistant en la fourniture d'une solution permettant de contrôler la température des tissus devant être protégés afin de limiter les effets de cavitation rencontrés pour des énergies acoustiques élevées comme cela est nécessaire dans le cadre de la thérapie, en particulier au niveau des interfaces et surtout à l'interface définie par la peau d'un mammifère à traiter, en particulier un être humain.

Tous ces problèmes techniques sont résolus pour la première fois par la présente invention d'une manière simultanée, simple, fiable, peu coûteuse, utilisable à l'échelle industrielle et médicale.

Ainsi, selon un premier aspect, la présente invention fournit un appareil de thérapie par ultrasons, comprenant au moins un dispositif de traitement comprenant au moins un élément transducteur piézoélectrique prévu pour réaliser au moins ladite thérapie en vue du traitement d'une cible à traiter, telle que des tissus pouvant se trouver à l'intérieur du corps d'un mammifère, en particulier d'un être humain, et des moyens de commande dudit dispositif pour réaliser ladite thérapie, ledit élément transducteur piézoélectrique étant prévu pour délivrer des ondes ultrasoniques focalisées en un point ou zone focale déterminant la zone tissulaire à soumettre à ladite thérapie, caractérisé en ce que les moyens de commande dudit dispositif permettent de commander l'émission alternée d'ondes ultrasoniques de deux types, le premier type dit thermique produisant sur les tissus un effet thermique prépondérant, et un deuxième type dit de cavitation produisant sur les tissus à traiter un effet de cavitation prépondérant.

Selon une variante de réalisation avantageuse, les moyens de commande précités commandent au dispositif de traitement, au moins en début du traitement, des ondes ultrasoniques thermiques.

Selon un autre mode de réalisation avantageux, les moyens de commande précités du dispositif de traitement commandent l'émission d'ondes ultrasoniques de cavitation après un laps de temps prédéterminé réglable pendant lequel des ondes ultrasoniques thermiques sont émises, en permettant ainsi de réaliser un préchauffage des tissus à traiter.

Selon un autre mode de réalisation particulier, les moyens de commande précités permettent de commander l'émission d'ondes ultrasoniques de cavitation simultanément à l'émission d'ondes ultrasoniques thermiques, en particulier après le laps de temps précité pendant lequel seules des ondes ultrasoniques thermiques sont émises.

Selon encore une variante de réalisation particulière, l'intensité acoustique des ondes ultrasoniques thermiques est inférieure au seuil de cavitation tandis que l'intensité acoustique des ondes ultrasoniques de cavitation est au moins égale au seuil de cavitation, lequel seuil de cavitation est fonction des tissus à traiter des mammifères.

Selon une autre variante de réalisation particulière, la fréquence des ondes ultrasoniques de cavitation est inférieure à la fréquence des ondes ultrasoniques thermiques.

Selon une autre variante de réalisation, les moyens de commande précités réalisent l'émission d'ondes ultrasoniques de cavitation comportant une composante négative dans l'amplitude, et de nature à déclencher la cavitation.

Selon un autre mode de réalisation particulier, les moyens de commande précités réalisent l'émission d'ondes ultrasoniques de cavitation d'une durée comprise entre environ 0,5 microseconde et environ 100 millisecondes, et de préférence comprise entre 0,5 microseconde et 50 microsecondes.

Selon encore un autre mode de réalisation particulier, les moyens de commande précités réalisent une émission d'ondes ultrasoniques de cavitation par impulsions successives, à une fréquence de répétition variant d'environ 1 Hz à 1 kiloHz, de préférence d'environ 10 Hz à 100 Hz.

Selon encore une variante de réalisation particulière, la durée du laps de temps prédéterminé réglable est comprise entre environ 100 millisecondes et environ 10 secondes.

Selon un autre mode de réalisation particulier, la durée totale du traitement de la zone tissulaire prédéterminée par le point ou la zone focale, par les ondes ultrasoniques précitées, est comprise entre 100 millisecondes et 10 secondes, cette durée totale incluant au moins une impulsion d'ondes ultrasoniques de cavitation.

Selon un autre de mode de réalisation particulièrement avantageux, l'appareil est caractérisé en ce qu'il comprend des moyens de déplacement du dispositif de traitement de manière à réaliser un traitement dit point par point, chaque point étant déterminé par le point ou zone focale précitée, de manière à couvrir l'ensemble du volume de la cible à traiter.

De préférence, les moyens de commande précités du dispositif de traitement sont commandés par une centrale de commande, par exemple comprenant un calculateur tel qu'un ordinateur, ou micro-ordinateur, celui-ci disposant de préférence d'un logiciel gérant les déplacements du dispositif de traitement en fonction du volume de la cible à traiter, avantageusement les données de volume ayant été acquises par des moyens d'imagerie associés.

Selon une variante de réalisation particulière, la centrale de commande précitée commande le déplacement des moyens de déplacement précités du dispositif de traitement de manière à réaliser le traitement des zones tissulaires de la cible les plus éloignées du dispositif de traitement jusqu'à aboutir aux zones tissulaires les plus proches du dispositif de traitement afin d'améliorer l'efficacité du traitement de la cible. Grâce à l'invention, on résout le problème du traitement des zones éloignées en les traitant en premier lieu pour éviter que la nécrose des zones proches fasse obstacle au traitement des zones éloignées.

Selon une variante de réalisation avantageuse, les moyens de commande précités assurent un temps de latence entre le traitement de deux points successifs de la cible à traiter afin de permettre une relaxation des tissus à traiter, de préférence ce temps de latence étant compris entre environ 1 seconde et 15 secondes, ce temps de latence étant avantageusement mis à profit pour réaliser le déplacement du dispositif de traitement d'un point de traitement à un autre.

Selon une autre variante de réalisation avantageuse, la centrale de commande précitée commande un déplacement des moyens de déplacement du dispositif de traitement précité d'une manière aléatoire tout en excluant les points déjà traités.

Selon une autre variante de réalisation avantageuse, la fréquence d'émission des ondes ultrasoniques de cavitation est comprise entre environ 500 kiloHz et 4 MHz, de préférence entre 500 kiloHz et 2 MHz, et encore mieux environ 1 MHz.

Selon une variante de réalisation particulière, la fréquence d'émission des ondes ultrasoniques thermiques est comprise entre environ 1 et 4 MHz, cette fréquence étant au moins égale à la fréquence des ondes ultrasoniques de cavitation.

Selon une autre variante de réalisation particulière, l'intensité acoustique des ondes ultrasoniques thermiques est inférieure à environ 150 W/cm² et l'intensité acoustique des ondes ultrasoniques de cavitation est au moins égale à environ 150 W/cm².

Selon un autre mode de réalisation avantageux de l'invention, les moyens de commande précités réalisent l'émission d'ondes ultrasoniques d'amplitude variable en fonction du temps, de préférence d'amplitude croissante au cours du temps, de manière que l'amplitude pendant une première période reste inférieure au seuil de cavitation, puis dans une deuxième période devienne supérieure au seuil de cavitation.

Aussi, selon un deuxième aspect, la présente invention fournit un appareil de thérapie par ultrasons, comprenant au moins un dispositif de traitement comprenant au moins un élément transducteur piézoélectrique prévu pour réaliser au moins ladite thérapie en vue de la destruction d'une cible à détruire telle que des tissus pouvant se trouver à l'intérieur du corps d'un mammifère, en particulier d'un être humain, et des moyens de commande dudit dispositif pour réaliser ladite thérapie, ledit élément transducteur piézoélectrique étant prévu pour délivrer des ondes ultrasoniques focalisées d'énergie acoustique élevée de thérapie en un point ou zone focale déterminant la zone tissulaire à soumettre à ladite thérapie, lesdites ondes ultrasoniques traversant des zones tissulaires situées à l'interface avec le dispositif de thérapie, caractérisé en ce qu'il comprend des moyens de refroidissement permettant de réaliser un refroidissement dans un domaine de température prédéterminé d'au moins lesdites zones tissulaires situées à l'interface avec le dispositif de thérapie, ce qui permet de protéger efficacement les zones tissulaires situées à ladite interface contre des effets de cavitation.

Selon encore un autre mode de réalisation particulièrement avantageux de l'invention, le dispositif de thérapie est extracorporel.

Selon un autre mode de réalisation de l'invention, le dispositif de thérapie est un dispositif endocavitaire permettant une thérapie par voie semi-invasive, en particulier ce dispositif endocavitaire peut être un dispositif endorectal ou bien un dispositif endo-urétral. Il peut encore s'agir d'un dispositif endo-oesophagien.

Selon encore un autre mode de réalisation avantageux de l'invention, on peut prévoir au moins un dispositif endocavitaire physiquement indépendant du dispositif de thérapie, pour le refroidissement de zones tissulaires éloignées du dispositif de thérapie et que l'on désire également protéger pendant ladite thérapie. Un tel dispositif endocavitaire est avantageusement prévu pour recevoir le même fluide réfrigérant que celui utilisé pour le dispositif de thérapie.

Selon encore un autre mode de réalisation de l'invention, indépendamment brevetable, on prévoit au moins un dispositif de mesure de la température des tissus situés à l'interface avec le dispositif de thérapie, des moyens de réception et de transmission des données de température transmises par le dispositif de mesure de température à une centrale de commande capable de modifier les instructions de commande de fonctionnement du dispositif de thérapie en fonction des données de température reçues. De préférence, les dispositifs de mesure de température des tissus de l'interface comprennent des capteurs sous forme de thermocouple, ou sous forme de feuille, en particulier de type PVDF qui présentent l'avantage de pouvoir se présenter sous forme de feuille(s) de très faible épaisseur, et que l'on peut disposer directement sur les zones tissulaires de l'interface en regard du dispositif de thérapie ou encore sur la face externe de la membrane renfermant le fluide de refroidissement et qui vient s'appliquer sur les tissus de l'interface. Egalement, la présence d'au moins un dispositif capteur de pression, en particulier de type PVDF, est avantageuse par le fait qu'elle peut permettre de mesurer la pression acoustique du champ ultrasonore émis par le dispositif de thérapie au niveau de ladite interface, ce qui permet de connaître très précisément l'intensité acoustique dans la zone focale, et cela en temps réel et de réguler la puissance électrique fournie à l'élément transducteur pour maintenir à une valeur constante la pression du champ acoustique ultrasonore au point focal F.

Un tel appareil de thérapie selon l'invention peut être utilisé ou appliqué à toutes les thérapies par ultrasons, de préférence focalisés, de toutes les tumeurs bénignes ou malignes connues à l'homme de l'art, et qu'elles soient externes ou internes. Les applications actuellement préférées sont le traitement des tumeurs bénignes et malignes du foie, de la prostate, du rein, du sein, de la peau, du cerveau et le traitement des varicosités ainsi que de l'oesophage. L'invention couvre ainsi également l'utilisation ou l'application de l'appareil de thérapie pour la fabrication d'un appareil pour le traitement de telles tumeurs bénignes ou malignes.

L'invention couvre encore un procédé de commande d'un appareil de thérapie ultrasonore, comme décrit à la revendication 28.

L'invention permet de combiner un traitement thermique des tissus avec un traitement par cavitation qui reste parfaitement maîtrisé spatialement. La combinaison de la cavitation au traitement thermique a pour effet de renforcer le potentiel destructeur du traitement, donc de limiter la durée des impulsions de traitement et d'éviter ainsi la diffusion thermique dans les tissus.

Dans le cas d'un traitement thérapeutique étendu à des lésions de volume supérieur à celui de la tache focale, les séquences de tir précédemment décrites sont réalisées point par point en déplaçant le point de focalisation, par des moyens de commande mécaniques ou électroniques associés, entre chaque tir, de telle sorte que le volume focal décrive la totalité du volume de la lésion.

Egalement, l'invention permet d'aboutir à un refroidissement particulièrement efficace des tissus situés dans la zone de l'interface avec le dispositif de thérapie, d'assurer également un refroidissement des éléments transducteurs piézoélectriques du dispositif de thérapie, et de manière inattendue, d'aboutir à une limitation des effets de cavitation des tissus de la zone d'interface et également dans le fluide de couplage, constitué ici par le fluide de refroidissement.

Ceci est par ailleurs réalisé de manière extrêmement simple puisque l'on peut utiliser de l'eau du robinet, de préférence dégazée, puisque celle-ci circule en circuit fermé et n'est jamais en contact avec le patient et que sa température peut être contrôlée de manière extrêmement simple par tous dispositifs de régulation de température bien connus à l'homme de l'art. D'autre part, l'eau présente aussi l'avantage d'absorber très faiblement les ultrasons et par conséquent de ne pas s'échauffer sous l'action du champ ultrasonore et donc de conserver sa capacité réfrigérante.

Un autre effet inattendu résultant de la limitation des effets de cavitation réside dans le fait qu'il est possible d'accroître la puissance des ondes acoustiques et donc de limiter le temps de traitement, ce qui permet encore de limiter les effets d'échauffement des tissus notamment par effet de diffusion.

D'autres buts, caractéristiques et avantages de l'invention apparaîtront clairement à la lumière de la description explicative qui va suivre faite en référence aux dessins annexés représentant trois modes de réalisation actuellement préférés de l'invention, donnés simplement à titre d'illustration et qui ne sauraient donc en aucune façon limiter la portée de l'invention.
Dans les dessins :
- la figure 1 représente schématiquement les organes essentiels d'un appareil de thérapie par ultrasons selon la présente invention ;
- la figure 2 représente le seuil des effets de cavitation et les zones d'effets thermiques seules ou d'effets thermiques plus effets de cavitation en fonction de l'intensité acoustique en ordonnées exprimée en watt par centimètre carré en fonction du temps en abscisses exprimé en milliseconde ;
- la figure 3 représente schématiquement le principe de destruction de structures tissulaires par abaissement du seuil de cavitation avec le dispositif de traitement représenté à la figure 1 avec mention de plusieurs interfaces ;
- la figure 4 représente la courbe d'évolution du seuil de cavitation en fonction de l'intensité acoustique en ordonnées exprimée en watt par centimètre carré ainsi que du temps en abscisses exprimé en milliseconde, avec mention des seuils de cavitation pour les interfaces de la figure 3 ;
- la figure 5 représente les courbes des ondes ultrasoniques thermiques et des ondes ultrasoniques de cavitation en fonction de l'amplitude des impulsions en ordonnées, au cours du temps en abscisses exprimé en milliseconde ;
- la figure 6 représente un autre mode de réalisation des ondes ultrasoniques dont l'amplitude est croissante au cours du temps pour présenter une première période où cette amplitude est inférieure au seuil de cavitation puis une deuxième période où cette amplitude est supérieure au seuil de cavitation ;
- la figure 7 représente schématiquement la fréquence des traitements point par point avec la durée de traitement de chaque point ;
- la figure 8 représente un schéma de principe d'un appareil de thérapie extracorporel selon la présente invention comportant des moyens de refroidissement des tissus situés à l'interface avec le dispositif de thérapie ; et
- la figure 9 représente une variante de réalisation de l'appareil de la figure 8 comportant de manière complémentaire une sonde endocavitaire de refroidissement pour assurer un refroidissement de zones tissulaires éloignées du dispositif de thérapie.

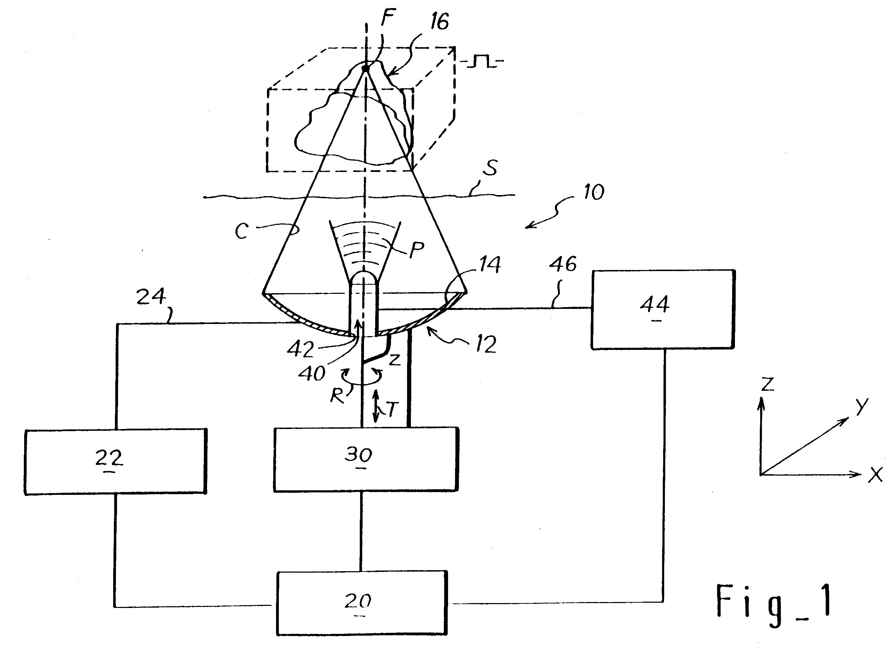

En référence à la figure 1, on a représenté un appareil de thérapie extracorporel selon l'invention représenté par le numéro de référence général 10. Cet appareil comprend au moins un dispositif de traitement extracorporel représenté par le numéro de référence général 12 comprenant au moins un élément transducteur 14 piézoélectrique prévu pour réaliser au moins ladite thérapie en vue du traitement d'une cible à traiter, telle que des tissus, représentée schématiquement par le numéro de référence général 16, pouvant se trouver à l'intérieur du corps d'un mammifère M, en particulier d'un être humain. La surface de la peau de ce mammifère est représentée par la lettre S. Cet appareil comprend aussi des moyens de commande tels que 20, 22 commandant le dispositif 12, comme cela est symbolisé par la liaison référencée 24. L'élément transducteur piézoélectrique 14 est de préférence prévu pour délivrer des ondes ultrasoniques focalisées en un point ou une zone focale F, le champ acoustique focalisé étant symbolisé par la lettre C. Le point de la zone focale F détermine naturellement la zone tissulaire à soumettre à ladite thérapie.

Cet appareil est caractérisé selon l'invention en ce qu'il comprend des moyens de commande 20, 22 dudit dispositif de traitement 12 prévus ou conçus pour faire délivrer par ledit dispositif de traitement 12 des ondes ultrasoniques 12 de deux types, un premier type dit thermique d'ondes ultrasoniques produisant sur les tissus à traiter 16 un effet thermique prépondérant, et un deuxième type dit de cavitation d'ondes ultrasoniques produisant sur les tissus 16 à traiter un effet de cavitation prépondérant.

Selon une variante de réalisation avantageuse, les moyens de commande 20, 22 commandent au dispositif de traitement 12 au moins en début de traitement, des ondes ultrasoniques thermiques.

Selon un autre inode de réalisation avantageux, les moyens de commande du dispositif de traitement commandent l'émission d'ondes ultrasoniques de cavitation après un laps de temps prédéterminé réglable pendant lequel les ondes ultrasoniques thermiques sont émises, en permettant ainsi de réaliser un préchauffage des tissus à traiter. Des moyens de commande précités peuvent avantageusement permettre de commander l'émission d'ondes ultrasoniques de cavitation simultanément à l'émission d'ondes ultrasoniques thermiques, et en particulier après le laps de temps précité pendant lequel seules des ondes ultrasoniques thermiques sont émises.

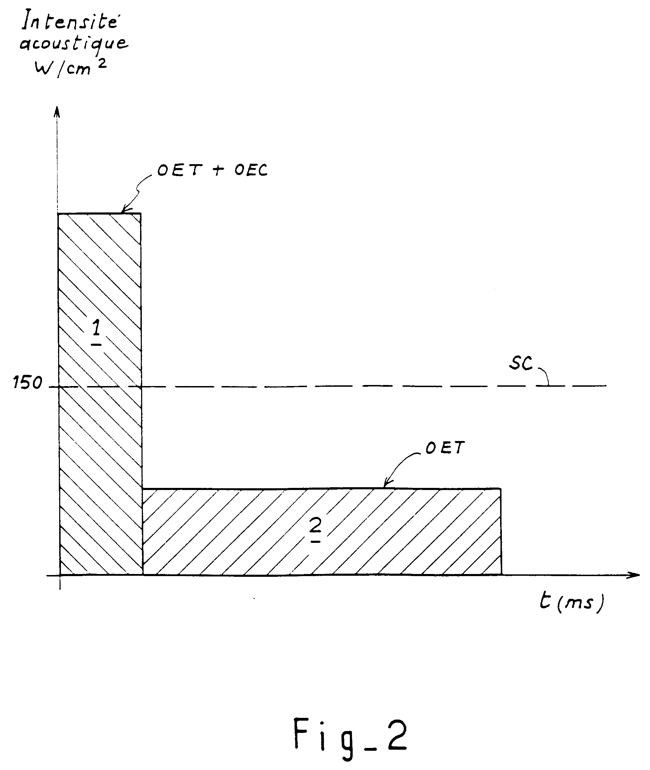

Selon encore une autre variante de réalisation particulière, l'intensité acoustique des ondes ultrasoniques thermiques est inférieure au seuil de cavitation au point focal F représenté en trait mixte à la figure 2 par les références SC, tandis que l'intensité acoustique des ondes ultrasoniques de cavitation est au moins égale au seuil de cavitation SC au point focal F, lequel seuil de cavitation SC au point focal F est fonction des tissus à traiter des mammifères.

Selon une autre variante de réalisation particulière, la fréquence des ondes ultrasoniques de cavitation émise par l'élément transducteur 14 en fonction des commandes des moyens 20, 22, est inférieure à la fréquence des ondes ultrasoniques thermiques.

Selon une autre variante de réalisation, les moyens de commande précités réalisent une émission d'ondes ultrasoniques de cavitation comportant une composante négative dans l'amplitude, et de nature à déclencher la cavitation.

Selon encore une autre variante de réalisation, l'intensité acoustique des ondes ultrasoniques thermiques est inférieure à environ 150 W/cm² et l'intensité acoustique des ondes ultrasoniques de cavitation est au moins égale à environ 150 W/cm2. La valeur de 150 W/cm², comme montré à la figure 2, représente le seuil de cavitation au point focal F des tissus d'une tumeur cancéreuse - du corps d'un mammifère, en particulier d'un être humain. A la figure 2, on a représenté une zone 1 d'intensité acoustique pour laquelle les ondes ultrasoniques ont une intensité acoustique allant au-delà du seuil de cavitation. Ces ondes ultrasoniques présentent donc une combinaison d'ondes acoustiques à effet thermique (OET) et des ondes ultrasoniques à effet de cavitation prépondérant (OEC). Par contre, dans la zone 2, l'intensité acoustique des ondes ultrasoniques étant bien inférieure au seuil de cavitation, les ondes ultrasoniques sont seulement des ondes ultrasoniques à effet thermique comme cela est bien compréhensible à la considération de la figure 2.

Selon une autre variante de réalisation particulière, la fréquence des ondes ultrasoniques de cavitation est inférieure à la fréquence des ondes ultrasoniques thermiques.

Selon une autre variante de réalisation, les moyens de commande précités réalisent l'émission d'ondes ultrasoniques de cavitation comportant une composante négative dans l'amplitude de nature à déclencher la cavitation.

Selon un autre mode de réalisation particulier, les moyens de commande 20, 22 réalisent une émission d'ondes ultrasoniques de cavitation d'une durée comprise entre environ 0,5 microseconde et environ 100 millisecondes, et de préférence comprise entre 0,5 microseconde et 50 microsecondes.

Selon encore un autre mode de réalisation particulier, les moyens de commande 20, 22 réalisent une émission d'ondes ultrasoniques de cavitation par impulsions successives, à une fréquence de répétition variant d'environ 1 Hz à 1 kiloHz, de préférence d'environ 10 Hz à 100 Hz.

Selon encore une variante de réalisation particulière, la durée du laps de temps prédéterminé réglable précité est comprise entre environ 100 millisecondes et environ 10 secondes.

Selon encore un autre mode de réalisation particulier, la durée totale du traitement de la zone tissulaire déterminée par le point ou la zone focale F, par les ondes ultrasoniques précitées, est comprise entre 100 millisecondes et 10 secondes, cette durée totale incluant au moins une impulsion d'ondes ultrasoniques de cavitation.

Selon un autre mode de réalisation particulièrement avantageux, l'appareil est caractérisé en ce qu'il comprend des moyens 30 de déplacement du dispositif de traitement 12 de manière à réaliser un traitement dit point par point, chaque point étant déterminé par le point ou zone focale F, de manière à couvrir l'ensemble du volume de la cible 16 à traiter.

De préférence, les moyens de commande précités 22 du dispositif de traitement 12 sont commandés par une centrale de commande 20, par exemple comprenant un calculateur tel qu'un ordinateur, ou micro-ordinateur, celui-ci disposant de préférence d'un logiciel gérant les déplacements du dispositif de traitement 12 en commandant les moyens de déplacement 30 de manière appropriée dans les trois directions dans l'espace X, Y et Z, en fonction du volume de la cible à traiter.

Avantageusement, il est également prévu un dispositif d'imagerie représenté par le numéro de référence général 40, tel qu'une sonde échographique ultrasonique par exemple disposée dans une ouverture centrale 42 du dispositif de traitement 12, ce dispositif d'imagerie 40 étant de préférence disposé coaxialement au dispositif de traitement focalisé 12 de manière à visualiser en permanence le point ou zone focale F. Ce dispositif d'imagerie 40, comme cela est bien connu, est avantageusement monté en rotation autour de son axe comme symbolisé par les flèches R et/ou en translation selon son axe comme symbolisé par la flèche T par des moyens de commande 44 avec une liaison 46. Ce dispositif d'imagerie comprend de préférence une sonde échographique ultrasonique du commerce réalisant une échographie de type B, c'est-à-dire à balayage dans un plan symbolisé schématiquement à la figure 1 et portant le numéro de référence P. Le dispositif d'imagerie 40 permet donc d'acquérir des données de volume de la cible 16 à traiter qui sont transmises à la centrale de commande 20 et traitées par le logiciel de cette centrale de commande. La centrale 20 commande, par les moyens 30, les déplacements du dispositif de traitement 12, ainsi que par les moyens 44 le déplacement de la sonde d'imagerie 40, en rotation et/ou également en translation.

Selon une variante de réalisation particulière, la centrale de commande 20 commande le déplacement des moyens de déplacement 30 du dispositif de traitement 12 et/ou de la sonde d'imagerie associée, c'est-à-dire solidaire en déplacement du dispositif de traitement 12, de manière à réaliser le traitement des zones tissulaires de la cible les plus éloignées du dispositif de traitement 12, comme représenté sur la figure 1, jusqu'à aboutir aux zones tissulaires les plus proches du dispositif de traitement 12 afin d'améliorer l'efficacité du traitement de la cible 16.

Selon une autre variante de réalisation avantageuse, la centrale de commande 20 commande un déplacement des moyens de déplacement 30 du dispositif de traitement 12 d'une manière aléatoire tout en excluant les points déjà traités.

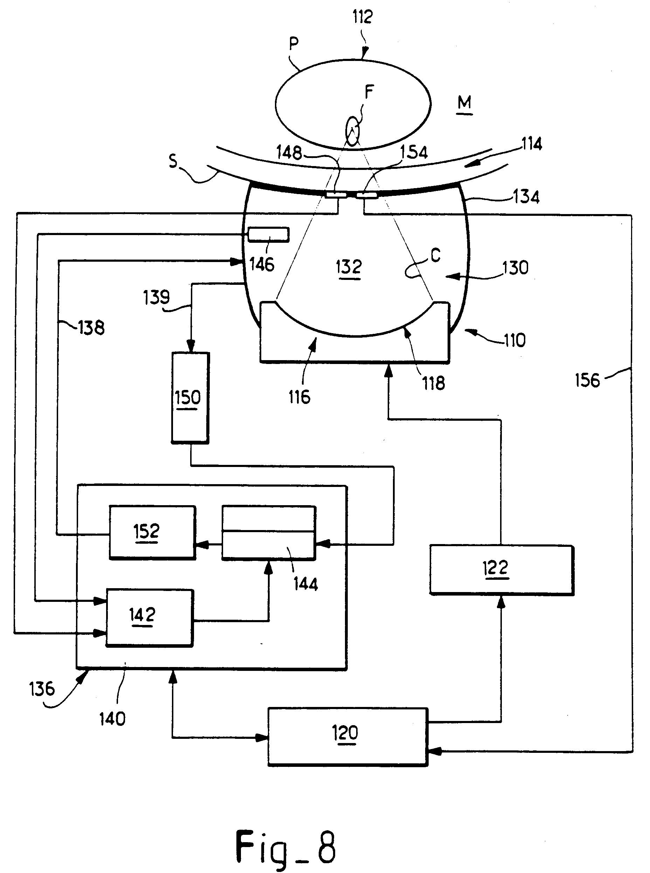

Selon une variante de réalisation avantageuse, les moyens de commande précités 22 assurent un temps de latence entre le traitement de deux points successifs de la cible à traiter afin de permettre une relaxation des tissus à traiter, de préférence le temps de latence étant compris entre environ 1 seconde et 15 secondes, ce temps de latence étant avantageusement mis à profit pour réaliser le déplacement du dispositif de traitement 12 d'un point de traitement à un autre, par une commande des moyens de déplacement 30 depuis la centrale de commande. En particulier, en référence à la figure 7, on a représenté la séquence de traitement point par point. Par exemple, le traitement du premier point de la première zone focale est dénommé F1 et le temps de traitement est désigné t_{F1}, le temps de latence qui suit est désigné t_{L1}, de sorte que le temps de traitement total du point formé par la zone focale F1 est t (total F1). Pour le point suivant numéro 2 formé par la zone focale F2, différente de F1, et qui est déterminé par la centrale de commande 20, le temps de traitement du point F2 est désigné Ft_{F2}, le temps de latence est désigné t_{L2} et le temps total du traitement t (total F2) et ainsi de suite pour les autres points.

Selon une autre variante de réalisation avantageuse, la fréquence d'émission des ondes ultrasoniques de cavitation est comprise entre environ 500 kiloHz et 4 MHz, de préférence entre 500 kiloHz et 2 MHz, et encore mieux environ 1 MHz.

Selon une variante de réalisation particulière, la fréquence d'émission des ondes ultrasoniques thermiques est comprise entre environ 2 et 4 MHz, cette fréquence étant au moins égale à la fréquence des ondes ultrasoniques de cavitation.

On conçoit ainsi qu'un tel appareil de thérapie selon l'invention peut être utilisé ou appliqué à toutes les thérapies par ultrasons, de préférence focalisés, de toutes les tumeurs bénignes ou malignes connues à l'homme de l'art, et qu'elles soient externes ou internes. Les applications actuellement préférées sont le traitement des tumeurs bénignes et malignes du foie, de la prostate, du rein, du sein, de la peau, du cerveau et le traitement des varicosités ainsi que de l'oesophage. L'invention couvre ainsi également l'utilisation ou l'application de l'appareil de thérapie tel que précédemment décrit et représenté aux dessins qui font partie intégrante de l'invention et donc de la présente description, pour la fabrication d'un appareil pour le traitement de telles tumeurs bénignes ou malignes.

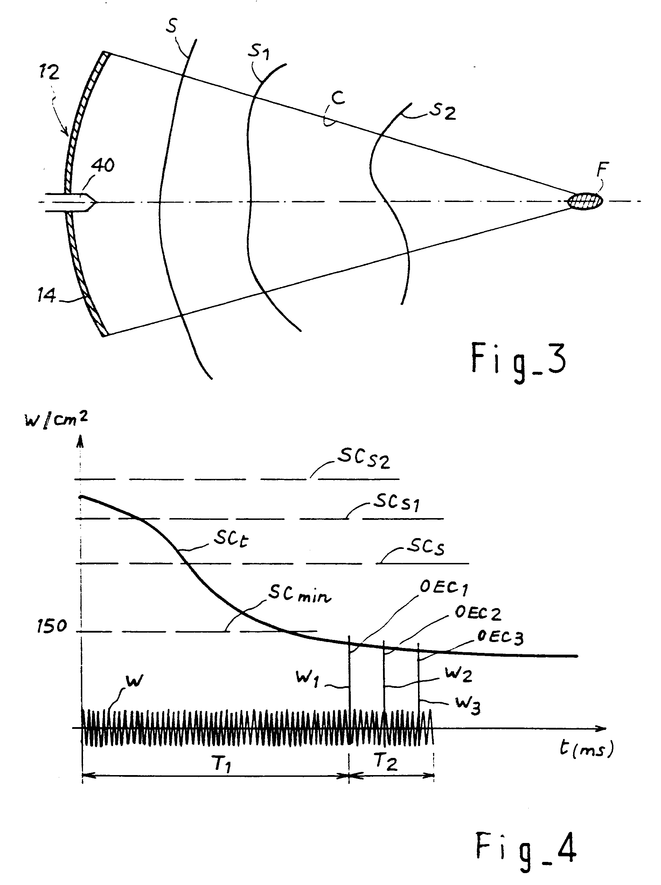

En référence à la figure 3, il a été mentionné les diverses interfaces (telles que la peau du patient S, S1 et S2 des interfaces internes au patient) et la zone focale F. Le dispositif de traitement de la figure 1 est référencé 12 et l'élément transducteur piézoélectrique 14 avec le dispositif d'imagerie 40.

En référence à la figure 4, on a représenté en pointillé respectivement le seuil de cavitation minimal SCₘᵢₙ à 150 W/cm²; le seuil de cavitation de l'interface SC_{S}, le seuil de cavitation à l'interface S1 SC_{S1} et le seuil de cavitation à l'interface S2 SC_{S2}, à la température ambiante.

La courbe SC_{T} représente la courbe du seuil de cavitation au point focal en fonction du temps, et en fonction de la température des tissus. Sous l'effet de l'augmentation de la température des tissus au point focal F, le seuil de cavitation au point focal SC_{T} diminue pour devenir inférieur aux seuils de cavitation SC_{M}, SC_{S} et SCₘᵢₙ. On a représenté par la référence W le signal électrique de commande délivré par les moyens de commande 22 à l'élément transducteur 14 au cours du temps, dont l'amplitude permet de délivrer des ondes ultrasoniques à effet thermique prépondérant et les signaux électriques W1, W2 et W3 de courte durée de quelques microsecondes à quelques millisecondes délivrés à l'élément transducteur 14, permettant de lui faire délivrer des ondes ultrasoniques à effet de cavitation prépondérant dont l'intensité passe au-dessus du seuil de cavitation SC au point focal à l'instant de délivrance de celle-ci, comme cela est observé sur la figure 4. Ces ondes ultrasoniques à effet de cavitation prépondérant peuvent se superposer aux ondes W, comme représenté.

On observera que les ondes de cavitation référencées OEC1, OEC2, OEC3 correspondant aux signaux W1, W2, W3 ne sont délivrées qu'après un laps de temps prédéterminé T₁ pendant lequel les signaux W permettent de délivrer des ondes à effet thermique prépondérant pour réaliser un préchauffage des tissus de la zone focale F afin d'abaisser le seuil de cavitation au point focal F de manière significative. Ceci constitue également une caractéristique technique particulière. préférentielle de la présente invention.

La période T₂ correspond à la période de délivrance des ondes ultrasoniques de cavitation. Comme il a été mentionné précédemment, la fréquence d'émission des ondes ultrasoniques de cavitation est comprise entre environ 500 kiloHz et 4 MHz, de préférence entre 500 kiloHz et 2 MHz, et encore mieux environ 1 MHz tandis que la fréquence d'émission des ondes ultrasoniques thermiques est comprise entre environ 1 et 4 MHz, cette fréquence étant au moins égale à la fréquence des ondes ultrasoniques de cavitation. Par ailleurs, la durée des ondes ultrasoniques de cavitation est comprise entre environ 0,5 microseconde et environ 100 millisecondes, de préférence comprise entre 0,5 microseconde et 50 microsecondes. Par ailleurs, comme cela ressort de la figure 7, entre les différents points de traitement F₁, F₂, F₃, F₄, on observe un temps de latence de préférence compris entre environ 1 seconde et 15 secondes, ce temps de latence étant avantageusement mis à profit pour réaliser le déplacement du dispositif de traitement 12 d'un point à un autre, ainsi que pour actionner les moyens de commande d'imagerie 44 par une liaison 46 des moyens d'imagerie 40.

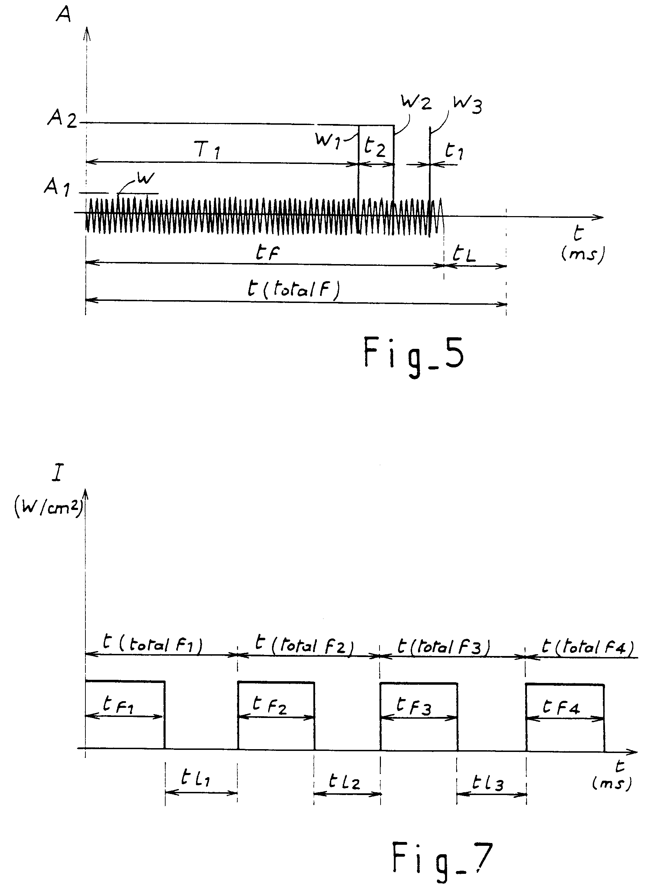

A la figure 5, on a représenté l'amplitude maximale A₁ des ondes ultrasoniques à effet thermique prépondérant W et l'amplitude maximale A₂ des ondes ultrasoniques à effet de cavitation prépondérant W₁, W₂, W₃. Il a également été représenté la période de temps T₁ pendant laquelle les ondes ultrasoniques de cavitation ne sont pas émises, cette période s'étalant avantageusement de 100 millisecondes à 10 secondes, il a également été représenté le temps de traitement par les ondes ultrasoniques d'un point de la zone focale F t_{F} ainsi que le temps t₁ représentant la durée de chaque impulsion d'ondes ultrasoniques de cavitation et le temps t₂ entre deux émissions successives d'ondes de cavitation, déterminant la fréquence de répétition des impulsions de cavitation. La fin du traitement est suivie éventuellement du temps de latence t_{L}.

Ceci détermine le temps total de traitement du point F t (total F). Ainsi, en référence à la figure 7, on retrouve ces données pour le traitement du premier point avec l'indice 1. Ainsi, au point 4 par exemple, celui-ci est référencé avec l'indice 4, etc.

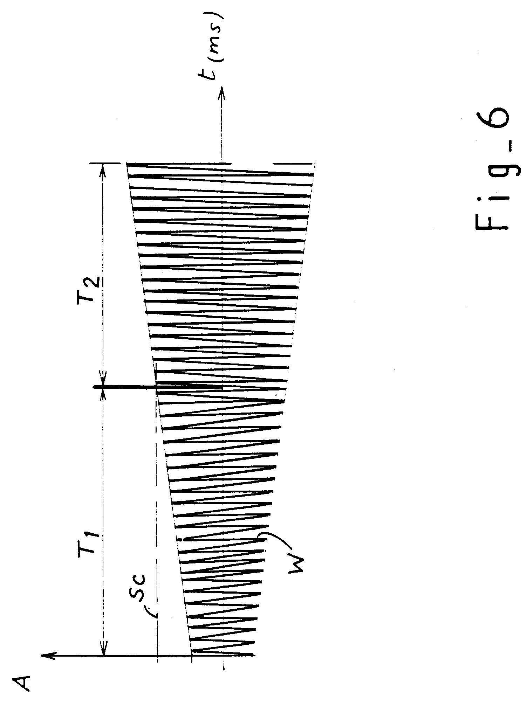

Enfin à la figure 6, on a représenté une autre variante de réalisation pour laquelle les moyens de commande 20, 22 réalisent l'émission d'ondes ultrasoniques d'amplitude variable en fonction du temps, de préférence d'amplitude croissante au cours du temps, de manière que l'amplitude pendant une première période T₁ reste inférieure au seuil de cavitation SC, puis dans une deuxième période T₂ devienne supérieure au seuil de cavitation SC.

En référence à la figure 8, on a représenté de manière schématique un autre mode de réalisation indépendamment brevetable éventuellement combinable avec celui des figures 1 à 7, d'un appareil de thérapie selon la présente invention représenté par le numéro de référence générale 110. Les tissus à traiter sont représentés par le numéro de référence générale 112, et la zone d'interface comportant notamment les tissus d'interface à préserver est représentée par le numéro de référence générale 114. Ces tissus d'interface particulièrement importants à préserver sont, par exemple, la peau d'un mammifère, de préférence un être humain.

L'appareil de thérapie selon la présente invention 110 comprend au moins un dispositif de traitement 116 comprenant au moins un élément transducteur piézoélectrique 118 prévu pour réaliser au moins la thérapie précitée en vue de la destruction d'une cible à détruire telle que les tissus 112 pouvant se trouver à l'intérieur du corps d'un mammifère M, en particulier d'un être humain. L'élément transducteur piézoélectrique 118 est prévu pour délivrer des doses ultrasoniques focalisées en un point ou zone focale F déterminant la zone tissulaire à soumettre à ladite thérapie, le champ de focalisation des ondes ultrasoniques étant schématisé par la référence C.

Des moyens de commande tels que 120, 122 du dispositif de traitement 116 pour réaliser la thérapie sont également prévus. Ces moyens de commande comprennent de préférence une centrale de commande 120 comprenant par exemple un calculateur, ordinateur ou micro-ordinateur, et des organes de commande mécanique et/ou électronique 122 du dispositif de traitement 116 et donc du ou des éléments transducteurs piézoélectriques 118 comme cela est bien connu à l'homme de l'art.

Selon ce mode de réalisation de l'invention l'appareil de thérapie 110 est caractérisé en ce qu'il comprend des moyens 130 de refroidissement permettant de réaliser un refroidissement dans un domaine de température prédéterminé d'au moins les zones tissulaires 114 situées à l'interface avec le dispositif de thérapie 116, ce qui permet de protéger efficacement les zones tissulaires situées à ladite interface 114.

Selon un mode de réalisation avantageux, les moyens de refroidissement 130 comprennent un fluide réfrigérant 132, de préférence un milieu liquide réfrigérant, en particulier de l'eau dégazée.

Selon un autre mode de réalisation particulièrement avantageux, le dispositif de thérapie 116 est pourvu d'une membrane en forme de poche, fixée de manière étanche sur le dispositif de traitement 116, comme cela est compréhensible pour un homme de l'art à partir de la considération de la figure 8. Cette membrane, fermée de manière étanche, est complètement remplie du fluide de refroidissement 132. Des moyens 136 de mise en circulation du fluide de refroidissement sont également prévus en vue de son renouvellement pour un maintien dans le domaine de température de refroidissement prévu.

Selon une variante de réalisation particulière, les moyens de refroidissement 130 assurent également un refroidissement de l'élément transducteur piézoélectrique 118, ce qui est généralement le cas lorsque la membrane 134 entoure extérieurement au moins l'élément transducteur 118 de manière que celui-ci baigne ou soit en contact permanent avec le fluide de refroidissement 132.

Les moyens de mise en circulation 136 comprennent par exemple un conduit 138 d'amenée de fluide de refroidissement depuis un appareillage 140 de thermostabilisation comprenant au moins un dispositif de régulation de température 142 commandant un groupe 144 de production de froid, par exemple un échangeur de chaleur, le dispositif 142 étant couplé à un ou plusieurs capteurs de température 146, 148 dont l'un tel que 146 peut être disposé dans le fluide de refroidissement 132 et l'autre tel que 148 peut être disposé extérieurement à la membrane 134 entre celle-ci et la surface S de la peau du mammifère M. Le fluide de refroidissement sort par un conduit 139 d'évacuation pour retourner au groupe 144 en passant éventuellement par un dispositif de dégazage 150. On peut naturellement prévoir des pompes de circulation telles que 152.

L'ensemble des moyens de refroidissement est avantageusement commandé par la centrale de commande 120.

Par ailleurs, on peut également prévoir un dispositif capteur de pression 154 également interposé extérieurement entre la membrane 134 et la surface S de la peau du mammifère M transmettant les données de pression par un élément conducteur correspondant 156 à la centrale de commande 120 pour que celle-ci modifie les ordres de commande des moyens de commande 122.

Selon un mode de réalisation particulièrement avantageux de l'invention, tel que représenté sur la figure 8, le dispositif de thérapie 110 est extracorporel.

Selon un autre mode de réalisation, non représenté, le dispositif de thérapie peut être un dispositif endocavitaire permettant une thérapie par voie semi-invasive, en particulier ce dispositif endocavitaire peut être un dispositif endorectal ou bien un dispositif endo-urétral. Il peut encore s'agir d'un dispositif endo-oesophagien. Etant donné que la réalisation d'un tel dispositif endocavitaire est bien connue à l'homme de l'art, aucune représentation dans les dessins n'apparaît nécessaire. On peut également se reporter à la demande antérieure des déposants WO-A-92/15253.

Les dispositifs de mesure de température des tissus tels que 148, comprennent avantageusement des capteurs sous forme de thermocouple, ou sous forme de feuille, en particulier de type PVDF qui présentent l'avantage de pouvoir se présenter sous forme de feuille(s) de très faible épaisseur, et que l'on peut ainsi disposer directement sur les zones tissulaires de l'interface en regard du dispositif de thérapie 116 ou encore sur la face externe de la membrane 134, comme représenté, cette membrane venant s'appliquer sur la surface S des tissus d'interface 114. Par ailleurs, ici, le capteur 154 qui est aussi avantageusement sous forme de feuille(s), en particulier de type PVDF, permet de mesurer la pression acoustique du champ ultrasonore émis par le dispositif de thérapie 116 au niveau de l'interface 114, ce qui permet de connaître très précisément l'intensité acoustique dans la zone focale, et cela en temps réel.

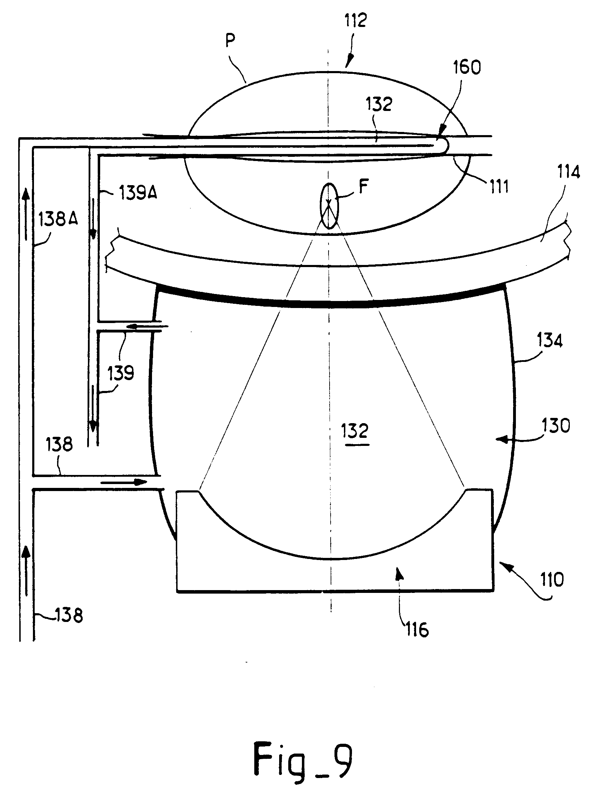

En référence à la figure 9, on a représenté une variante de réalisation de l'appareil de la figure 8 dans lequel il a été prévu au moins un dispositif endocavitaire 160 physiquement indépendant du dispositif de thérapie 116, pour le refroidissement de zones tissulaires éloignées du dispositif de thérapie 116 et que l'on désire également protéger pendant ladite thérapie. Ce dispositif endocavitaire 160 est avantageusement prévu pour recevoir le même fluide réfrigérant 132 que celui utilisé pour le dispositif de thérapie 116. Ici, pour un traitement de la prostate P, on a représenté schématiquement l'urètre U et le dispositif endocavitaire 160 est un dispositif endo-urétral. Ce dispositif endocavitaire 160 comprend un conduit 138A d'amenée de fluide réfrigérant 132 dérivant du conduit 138 de la figure 8 et un conduit d'évacuation 139A dérivant du conduit 139 d'évacuation de la figure 8, ce qui représente une modification extrêmement mineure de l'appareillage dans son ensemble.

On peut également prévoir que la sonde endocavitaire 160 soit pourvue de dispositifs de mesure de température permettant de contrôler la température atteinte dans l'urètre U, en constituant ainsi une sécurité complémentaire du traitement.

Le fonctionnement de cet appareil selon les figures 8 et 9 est immédiatement apparent à l'homme de l'art à partir de la description précédente.

On précisera à ce sujet que comme élément transducteur piézoélectrique focalisé préféré on utilisera un élément transducteur ayant une ouverture 1 et fonctionnant à une fréquence de 1 MHz. Pour ce type d'élément transducteur, le volume de la zone focale F aura typiquement une forme elliptique comme représentée ayant 10 mm de grand axe et 2 mm de petit axe.

On constate ici que le volume de la zone focale F est très petit par rapport à la prostate P qui définit le volume total de la zone tissulaire 112 à soumettre à la thérapie ultrasonique.

Compte tenu que le volume de la zone focale F est très petit par rapport au volume total des tissus à traiter, pendant le traitement, il suffira de déplacer le dispositif de traitement 116 pour réaliser un traitement dit point par point pour parcourir la totalité du volume de la lésion à traiter.

On comprend qu'en raison de la chaleur particulièrement intense créée dans le volume focal F il va se produire une diffusion thermique à travers les tissus qui peut s'étendre bien au-delà de la lésion à détruire et envahir en particulier la zone tissulaire à préserver telle que la zone 114. Ceci est particulièrement le cas lorsque le volume focal F est placé à proximité de la zone tissulaire 14 à préserver comme représenté aux figures 1 à 9 ;

Cette protection est aussi assurée par le refroidissement d'au moins la zone tissulaire 114 (figure 8), éventuellement avec la zone de l'urètre U (figure 9) grâce à la présence de la membrane 134 placée au contact du tissu 114 et éventuellement grâce à la présence de la sonde endocavitaire complémentaire 162.

La membrane 134 se caractérise par sa transparence au champ acoustique et sa capacité de conductivité thermique. Pour cela on choisit avantageusement un matériau adapté qui peut être du latex ou du silicone. L'épaisseur de la membrane dans la zone où passe le champ acoustique est de préférence réduite au minimum. Elle peut varier de quelques micromètres à quelques millimètres selon l'application envisagée (extracorporelle ou endocavitaire).

Grâce à la gestion assurée par la centrale de commande 120 et le dispositif de thermostatisation 140, le fluide réfrigérant 132 est réfrigéré à une température prédéterminée inférieure à la température du corps du mammifère, en particulier inférieure à 37°C et mieux inférieure à 35°C et encore mieux inférieure à 30°C. Une plage de températures de refroidissement particulièrement intéressante se situera entre 4°C et 30°C, mieux entre 15°C et 25°C.

Il est à noter que les capteurs de température et/ou de pression 148, 154, sous forme de feuille(s) d'épaisseur très fine sont transparents au champ acoustique et ne causent en pratique aucune perturbation. Etant donné que ces capteurs sont sensibles à la pression, ils permettent de capter la pression du champ acoustique fournie par l'élément transducteur focalisé 118. Cette information de pression est transmise à la centrale de commande 120 et permet de commander les moyens de commande 122 pour notamment réguler la puissance électrique fournie à l'élément transducteur acoustique ultrasonore.

Naturellement, pour réaliser la thérapie, il est possible d'enduire la peau du patient avec un agent de couplage tel que de l'huile silicone.

On peut également prévoir un dispositif de repérage, tel que le dispositif 40, figure 1, permettant un positionnement précis de la zone focale F de l'élément transducteur focalisé 118 en regard de la lésion à détruire.

L'alternance de tir ultrasonique et de déplacement de l'élément transducteur 118 permet de faire parcourir au volume focal F la totalité du volume de la lésion à détruire.

En fonction de la profondeur de la lésion à détruire on choisit une puissance ultrasonore particulière. Cette puissance ultrasonore est contrôlée et régulée au cours des tirs successifs par le dispositif capteur de pression 154.

Dès les premiers tirs, le capteur de température 148 mesure la température atteinte par les tissus à préserver 114, dans ce cas la peau du patient, et la transmet au dispositif de régulation de température 136. Ce dispositif agit sur le groupe 144 de production de froid pour maintenir constante à une valeur déterminée la température des tissus à préserver pour éviter ou limiter les effets de cavitation résultant des ondes ultrasoniques d'énergie acoustique élevée de thérapie. Cette régulation est obtenue en refroidissant plus ou moins la température du fluide réfrigérant 132, de préférence un liquide tel que de l'eau du robinet dégazé.

On conçoit ainsi que l'invention permet d'aboutir aux avantages techniques déterminants précédemment énoncés d'une manière simple, sûre, efficace d'un point de vue thérapeutique et avec une grande versatilité pour s'adapter à tous les types de lésions à traiter. L'invention comprend naturellement tous les moyens constituant des équivalents techniques des moyens faisant l'objet des revendications suivantes.

## Revendications

1. Appareil de thérapie par ultrasons, comprenant au moins un dispositif de traitement (12) comprenant au moins un élément transducteur piézoélectrique (14) prévu pour réaliser au moins ladite thérapie en vue de la destruction d'une cible (16) à détruire telle que des tissus pouvant se trouver à l'intérieur d'un mammifère, en particulier d'un être humain, et des moyens de commande dudit dispositif (12) pour réaliser ladite thérapie, ledit élément transducteur piézoélectrique (14) étant prévu pour délivrer des ondes ultrasoniques focalisées en un point ou zone focale (F) déterminant la zone tissulaire à soumettre à ladite thérapie, caractérisé en ce que les moyens de commande (20, 22) dudit dispositif (12) permettent de commander l'émission alternée d'ondes ultrasoniques de deux types, le premier type dit thermique produisant sur les tissus (16) à traiter un effet thermique prépondérant, et un deuxième type dit de cavitation produisant sur les tissus à traiter un effet de cavitation prépondérant.

2. Appareil selon la revendication 1, caractérisé en ce que les moyens de commande (20, 22) précités commandent au dispositif de traitement (12), au moins en début de traitement, des ondes ultrasoniques thermiques.

3. Appareil selon la revendication 1 ou 2, caractérisé en ce que les moyens de commande (20, 22) précités du dispositif de traitement (12) commandent l'émission d'ondes ultrasoniques de cavitation après un laps de temps prédéterminé réglable permettant de réaliser un préchauffage des tissus à traiter.

4. Appareil selon l'une des revendications précédentes, caractérisé en ce que les moyens de commande (20, 22) précités permettent de commander l'émission d'ondes ultrasoniques de cavitation simultanément à l'émission d'ondes ultrasoniques thermiques.

5. Appareil selon l'une des revendications précédentes, caractérisé en ce que l'intensité acoustique des ondes ultrasoniques thermiques est inférieure au seuil de cavitation tandis que l'intensité acoustique des ondes ultrasoniques de cavitation est au moins égale au seuil de cavitation, lequel seuil de cavitation est fonction des tissus à traiter du mammifère.

6. Appareil selon l'une des revendications précédentes, caractérisé en ce que la fréquence des ondes ultrasoniques de cavitation est inférieure à la fréquence des ondes ultrasoniques thermiques.

7. Appareil selon l'une des revendications précédentes, caractérisé en ce que des moyens de commande (20, 22) réalisent l'émission d'ondes ultrasoniques de cavitation comportant une composante négative dans l'amplitude, et de nature à déclencher la cavitation.

8. Appareil selon l'une des revendications précédentes, caractérisé en ce que les moyens de commande (20, 22) précités réalisent l'émission d'ondes ultrasoniques de cavitation d'une durée comprise entre environ 0,5 microseconde et environ 100 millisecondes, et de préférence comprise entre 0,5 microseconde et 50 microsecondes.

9. Appareil selon l'une des revendications précédentes, caractérisé en ce que les moyens de commande (20, 22) précités réalisent l'émission d'ondes ultrasoniques de cavitation par impulsions successives, à une fréquence de répétition variant d'environ 1 Hz à 1 kiloHz, de préférence d'environ 10 Hz à 100 Hz.

10. Appareil selon l'une des revendications 3 à 9, caractérisé en ce que la durée du laps de temps prédéterminé réglable est comprise entre environ 100 millisecondes et environ 10 secondes.

11. Appareil selon l'une des revendications précédentes, caractérisé en ce que la durée totale du traitement de la zone tissulaire déterminée par le point ou la zone focale, par les ondes ultrasoniques précitées, est comprise entre 100 millisecondes et 10 secondes, cette durée totale incluant au moins une impulsion d'ondes ultrasoniques de cavitation.

12. Appareil selon l'une des revendications 1 à 11, caractérisé en ce qu'il comprend des moyens de déplacement (22) du dispositif de traitement de manière à réaliser un traitement dit point par point, chaque point étant déterminé par le point ou zone focale précitée, de manière à couvrir l'ensemble du volume de la cible à traiter.

13. Appareil selon la revendication 12, caractérisé en ce que les moyens de déplacement (22) précités du dispositif de traitement (12) sont commandés par une centrale de commande (20), par exemple comprenant un calculateur tel qu'un ordinateur, ou micro-ordinateur, celui-ci disposant de préférence d'un logiciel gérant les déplacement du dispositif de traitement (12) en fonction du volume de la cible à traiter, avantageusement les données de volume ayant été acquises par des moyens d'imagerie (40) associés ayant des moyens de commande d'imagerie particuliers (44) commandés par la centrale de commande (20) et de préférence des moyens de déplacement (30), en particulier en translation et/ou en rotation.

14. Appareil selon la revendication 13, caractérisé en ce que la centrale de commande (20) commande le déplacement des moyens de déplacement (22) précités du dispositif de traitement (12) de manière à réaliser le traitement des zones tissulaires de la cible (16) les plus éloignées du dispositif de traitement (12) jusqu'à aboutir aux zones tissulaires les plus proches du dispositif de traitement (12) afin d'améliorer l'efficacité de traitement de la cible.

15. Appareil selon l'une des revendications précédentes, caractérisé en ce que les moyens de commande (20, 22) précités assurent un temps de latence entre le traitement de deux points successifs de la cible à traiter afin de permettre une relaxation des tissus à traiter, de préférence ce temps de latence étant compris entre environ 1 seconde et 15 secondes, ce temps de latence est avantageusement mis à profit pour réaliser le déplacement du dispositif de traitement d'un point de traitement à un autre.

16. Appareil selon l'une des revendications précédentes, caractérisé en ce que la centrale de commande (20) commande un déplacement des moyens de déplacement (22) du dispositif de traitement (12) précité d'une manière aléatoire tout en excluant les points déjà traités.

17. Appareil selon l'une des revendications précédentes, caractérisé en ce que la fréquence d'émission des ondes ultrasoniques de cavitation est comprise entre environ 500 kiloHz et 4 MHz, de préférence entre 500 kiloHz et 2 MHz, et encore mieux environ 1 MHz.

18. Appareil selon l'une des revendications précédentes, caractérisé en ce que la fréquence d'émission des ondes ultrasoniques thermiques est comprise entre environ 1 et 4 MHz, cette fréquence étant au moins égale à la fréquence des ondes ultrasoniques de cavitation.

19. Appareil selon l'une des revendications précédentes, caractérisé en ce que l'intensité acoustique des ondes ultrasoniques thermiques est inférieure à environ 150 W/cm² et l'intensité acoustique des ondes ultrasoniques de cavitation est au moins égale à environ 150 W/cm².

20. Appareil selon l'une des revendications précédentes, caractérisé en ce que les moyens de commande (20, 22) précités réalisent l'émission d'ondes ultrasoniques d'amplitude variable en fonction du temps, de préférence d'amplitude croissante au cours du temps, de manière que l'amplitude pendant une première période (T₁, figure 6) restc inférieure au seuil de cavitation (SC_{T}), puis dans une deuxième période (T₂, figure 6) deviennent supérieure au seuil de cavitation (SC_{T}).

21. Appareil de thérapie par ultrasons (110) selon l'une des revendications 1 à 20, comprenant au moins un dispositif de traitement (116) prévu pour réaliser au moins ladite thérapie en vue de la destruction d'une cible à détruire (112) telle que des tissus (P) pouvant se trouver à l'intérieur du corps d'un mammifère (M), en particulier d'un être humain, et des moyens de commande (120, 122) dudit dispositif (116), pour réaliser ladite thérapie, ledit élément transducteur (118) piézoélectrique étant prévu pour délivrer des ondes ultrasoniques focalisées d'énergie acoustique élevée de thérapie en un point ou zone focale (F) déterminant la zone tissulaire (P) à soumettre à ladite thérapie, lesdites ondes ultrasoniques traversant des zones tissulaires (114) situées à l'interface avec le dispositif de thérapie, caractérisé en ce qu'il comprend des moyens de refroidissement (130), avantageusement comprenant un fluide réfrigérant (132), de préférence un milieu liquide réfrigérant, tel que l'eau, permettant de réaliser un refroidissement dans un domaine de température prédéterminé d'au moins les zones tissulaires (114) situées à l'interface avec le dispositif de thérapie (116), ce qui permet de protéger efficacement les zones tissulaires situées à ladite interface contre des effets de cavitation.

22. Appareil selon l'une des revendications 1 à 21, caractérisé en ce que le dispositif de thérapie (12, 116) est extracorporel.

23. Appareil selon l'une des revendications 1 à 21, caractérisé en ce que le dispositif de thérapie (116) est un dispositif endocavitaire permettant une thérapie par vole semi-invasive, en particulier ce dispositif endocavitaire est un dispositif endorectal ou endo-urétral ou encore oesophagien.

24. Appareil selon l'une des revendications précédentes, caractérisé en ce qu'il comprend au moins un dispositif endocavitaire (160) physiquement indépendant du dispositif de thérapie (12; 116), pour le refroidissement de zones tissulaires (U) éloignées du-dispositif de thérapie et que l'on désire également protéger pendant ladite thérapie.

25. Appareil selon l'une des revendications 1 à 24, caractérisé en ce qu'il comprend au moins un dispositif de mesure de température (148) des tissus (114) situé à l'interface avec le dispositif de thérapie (12; 116), des moyens de réception (142) et de transmission des données de température transmises par le dispositif de mesure de température à une centrale de commande (120) capable de modifier les instructions de commande de fonctionnement du dispositif de thérapie en fonction des données de température reçues.

26. Appareil selon la revendication 25, caractérisé en ce que le dispositif de mesure de température (148) comprend des capteurs sous forme de thermocouple ou sous forme de feuille, en particulier de type PVDF pouvant être disposés directement sur les zones tissulaires de l'interface en regard du dispositif de thérapie ou encore sur la face extrême de la membrane (134) renfermant le fluide de refroidissement (132) et qui vient s'appliquer sur les tissus de l'interface (114), de préférence au moins un dispositif capteur de pression, en particulier de type PVDF, permettant de mesurer la pression acoustique du champ ultrasonore émis par le dispositif de thérapie (116) au niveau de l'interface (114) afin de réguler la puissance électrique fournie à l'élément transducteur (118) pour maintenir à une valeur constante la pression du champ acoustique ultrasonore au point focal (F).

27. Appareil selon l'une des revendications précédentes, caractérisé en ce qu'il est utilisé ou appliqué à toutes les thérapies par ultrasons, de préférence localisés, de toutes les tumeurs bénignes ou malignes externes ou internes, de préférence pour le traitement des tumeurs bénignes et malignes du foie, de la prostate, du rein, du sein, de la peau, du cerveau et le traitement des varicosités ainsi que de l'oesophage.

28. Procédé de commande d'un appareil de thérapie ultrasonore, présentant au moins un élément transducteur piézoélectrique (14) et des moyens de commande dudit ou desdits éléments transducteurs (14) pour commander l'émission par ledit ou lesdits transducteurs d'ondes ultrasonores de thérapie vers des tissus à traiter, caractérisé en ce qu'il comprend les étapes successives ou simultanées suivantes:
- commander l'émission par ledit ou lesdits transducteurs d'ondes ultrasonores de thérapie présentant un effet thermique prépondérant dans les tissus à traiter;
- commander l'émission par ledit ou lesdits transducteurs d'ondes ultrasonores de thérapie présentant un effet de cavitation prépondérant dans les tissus à traiter.

## Claims

1. Apparatus for performing therapy using ultrasound, comprising at least one treatment device (12) comprising at least one piezoelectric transducer element (14) designed to provide at least said therapy for the purpose of destroying a target (16) to be destroyed such as tissue which may be located inside the body of a mammal, in particular a human being, and control means for said device (12) in order to carry out said therapy, said piezoelectric transducer element (14) being designed to supply ultrasonic waves focused onto a focal point or region (F) determining the tissue zone to be submitted to said therapy, characterized in that it comprises control means (20,22) for said device (12) designed to cause said treatment device (12) to supply ultrasonic waves of two types, the first type, referred to herein as thermal waves, producing a predominantly thermal effect on the tissues (16) to be treated, and a second type referred to herein as cavitation waves producing a predominantly cavitation effect on said tissues to be treated.

2. Apparatus according to claim 1, characterized in that the said control means (20, 22) control within said treatment device (12), at least at the beginning of said treatment, thermal ultrasonic waves.

3. Apparatus according to claim 1 or 2, characterized in that the said control means (20, 22) for the treatment device (12) control the transmission of cavitation ultrasonic waves after an adjustable predetermined time interval allowing pre-heating of the tissue to be treated to be obtained.

4. Apparatus according to one of the preceding claims, characterized in that the said control means (20, 22) enable the transmission of cavitation ultrasonic waves to be controlled simultaneously with the transmission of thermal ultrasonic waves.

5. Apparatus according to one of the preceding claim, characterized in that the acoustic power of said thermal ultrasonic waves is lower than the cavitation threshold whereas the acoustic power of said cavitation ultrasonic waves is at least equal to the cavitation threshold, said cavitation threshold being a function of the tissue of the mammal to be treated.

6. Apparatus according to one of the preceding claims, characterized in that the frequency of said cavitation ultrasonic waves is lower than the frequency of said thermal ultrasonic waves.

7. Apparatus according to one of the preceding claims, characterized in that said control means (20, 22) provide for transmission of cavitation ultrasound waves including a negative component of the amplitude thereof of a nature to initiate cavitation.

8. Apparatus according to one of the preceding claims, characterized in that the said control means (20, 22) provide the transmission of cavitation ultrasound waves for a duration comprised between about 0.5 microseconds and about 100 milliseconds, and preferably comprised between 0.5 microseconds and 50 microseconds.

9. Apparatus according to one of the preceding claims, characterized in that the said control means (20, 22) provide transmission of cavitation ultrasound waves by successive pulses, the repetition frequency of which varies from about 1 Hz to 1 KHz, preferably from about 10 Hz to 100 Hz.

10. Apparatus according to one of claims 3 to 9, characterized in that the duration of said adjustable predetermined time interval is comprised between about 100 milliseconds and about 10 seconds.

11. Apparatus according to one of the preceding claims, characterized in that the total duration of treatment of the tissue region determined by the focal point or region by means of the said ultrasound waves is comprised between 100 milliseconds and 10 seconds, this total duration including at least one pulse of cavitation ultrasound waves.

12. Apparatus according to one of claims 1 to 11, characterized in that it comprises means (22) for displacing said treatment device in order to perform point-by-point treatment, each of said points being determined by the said focal point or region, in order to cover the whole volume of the target to be treated.

13. Apparatus according to claim 12, characterized in that the said displacement means (22) of the treatment device (12) are controlled by a control unit (20), comprising for example calculating means such a computer or a micro-computer, the latter being preferably provided with software managing the displacement of said treatment device (12) as a function of the volume of the target to be treated, volume data having advantageously been acquired by imaging means (40) associated therewith having special imaging control means (44) controlled by said control unit (20), and preferably displacement means (30), in particular for translatory and/or rotational displacement.

14. Apparatus according to claim 13, characterized in that said control unit (20) controls the displacement of said displacing means (22) of said treatment device (12) in order to carry out treatment of the tissue regions of said target (16) which are most remote from said treatment device (12) up to the tissue regions that are closest to said treatment device (12) in order to improve the effectiveness of treatment of said target.

15. Apparatus according to one of the preceding claims, characterized in that said control means (20, 22) provide a latency period between the treatment of two successive points on the target to be treated in order to allow said tissue being treated to relax, said latency period being preferably comprised between about 1 second and 15 seconds, said latency period being advantageously employed for carrying out the displacement of the treatment device from one treatment point to another.

16. Apparatus according to one of the preceding claims, characterized in that said control unit (20) controls a displacement of said displacement means (22) of said treatment device (12) in a random manner while nevertheless excluding points that have already been treated.

17. Apparatus according to one of the preceding claims, characterized in that the frequency of transmission of said cavitation ultrasound waves is comprised between about 500 KHz and 4 MHz, preferably between 500 KHz and 2 MHz, and even more preferably is about 1 MHz.

18. Apparatus according to one of the preceding claims, characterized in that the frequency of transmission of said thermal ultrasound waves is comprised between about 1 and 4 MHz, said frequency being at least equal to the frequency of said cavitation ultrasound waves.

19. Apparatus according to one of the preceding claims, characterized in that the acoustic power of said thermal ultrasound waves is lower than about 150 W/cm², and the acoustic power of said cavitation ultrasound waves is at least equal to about 150 W/cm².

20. Apparatus according to one of the preceding claims, characterized in that the said control means (20, 22) provide transmission of ultrasound waves of an amplitude that varies as a function of time, said amplitude preferably increasing with the passage of time, whereby the amplitude over a first period (T₁, FIG. 6) remains below a cavitation threshold (SC), then, in a second period (T₂ FIG. 6) becomes higher than said cavitation threshold (SC_{T}).

21. An apparatus for providing therapy by ultrasound (110), preferably according to one of claims 1 to 20, comprising at least one treatment device (116) designed to provide at least said therapy for the purpose of destroying a target (112) to be destroyed such as tissue (P) which may be located inside the body of a mammal (M), in particular a human being, and control means (120, 122) for said device (116) in order to carry out said therapy, said piezoelectric transducer element (118) being designed to supply ultrasonic high energy acoustic waves focused onto a focal point or region (F) determining the tissue zone (P) to be submitted to said therapy, said ultrasound waves passing through tissue regions (114) located at the interface with said therapy device, characterized in that it comprises cooling means (130) advantageously comprising a refrigerating fluid (132), preferably a liquid refrigerating medium, such as water, allowing cooling to be performed in a predetermined temperature range, of at least the tissue regions (114) located at the interface with said therapy device (116), allowing the tissue regions located at said interface to be efficiently protected against cavitation effects.

22. Apparatus according to one of claims 1 to 21, characterized in that said therapy device (12; 116) is extracorporeal.

23. Apparatus according to one of claims 1 to 21, characterized in that said therapy device (116) is an endo-cavitary device allowing therapy by semi-invasive treatment to be achieved, said endo-cavitary device being in particular an endo-rectal or endo-urethral or oesophagal device.

24. Apparatus according to one of the preceding claims, characterized in that it comprises at least one endo-cavitary device (116) physically independent of said therapy device (12; 116) for cooling tissue regions (U) remote from said therapy device and which it is also desired to protect during said therapy.

25. Apparatus according to one of claim 1 to 24, characterized in that it comprises at least one temperature measuring device (148) for the tissue (114) located at the interface with said therapy device (12, 116), means for receiving (142) and transmitting temperature data transmitted by the temperature measuring device to a control unit (120) capable of modifying the instructions controlling the operation of said therapy device as a function of the temperature data received.

26. Apparatus according to claim 25, characterized in that the temperature measuring device (148) comprises sensors in the form of a thermocouple or in sheet-form, in particular of the PVDF type, able to be arranged directly on the tissue regions of the interface facing said therapy device or, yet again, on the outer face of the membrane (134) enclosing said refigerating or cooling fluid (132) and designed for application to the tissue of the interface (114), at least one pressure sensor, in particular of the PVDF type preferably allowing the acoustic pressure of the ultrasound field delivered by the therapy device (116) to be measured at said interface (114) in order to regulate the electrical power supplied to said transducer element (118) so as to maintain the ultrasound acoustic field pressure at said focal point (F) at a constant value.

27. Apparatus according to one of the preceding claims, characterized in that it is used with, or applied to, all types of therapy by ultrasound, preferably localized, of all benign or malignant external or internal tumors, and preferably for the treatment of benign and malignant tumors of the liver, of the prostate, of the kidney, of the breast, of the skin, of the brain and for the treatment of varicose states and of the oesophagous.

28. A method for controlling ultrasound therapy apparatus having at least one piezoelectric transducer element (14) and means for controlling said one or plurality of transducer elements, for controlling delivery of ultrasound therapy waves by said transducers to the region to be treated, characterized in that it comprises the successive or simultaneous steps of:
- controlling delivery of ultrasound therapy waves by said ultrasound wave transducer or transducers having a predominantly thermal effect on the tissues to be treated;
- controlling delivery of ultrasound therapy waves by said ultrasound wave transducer or transducers having a predominantly cavitation effect on said tissues to be treated.

## Patentansprüche

1. Ultraschalltherapievorrichtung umfassend zumindest eine Behandlungsanordnung (12) mit zumindest einem piezoelektrischen Transducerelement (14), welches vorgesehen ist zum Durchführen zumindest der Therapie, angesichts der Zerstörung eines zu zerstörenden Zieles (16), wie Gewebe, welche sich im Inneren eines Säugetieres, insbesondere einem menschlichen Lebewesen befinden können, und eine Steuereinrichtung der Anordnung (12) zum Durchführen der Therapie, wobei das piezoelektrische Transducerelement (14) vorgesehen ist zum Liefern von Ultraschallwellen fokussiert in einem Punkt oder einer fokalen Zone (F), welche den Gewebebereich definiert, welcher mit der Therapie zu behandeln ist, dadurch gekennzeichnet, daß die Steuereinrichtung (20, 22) der Anordnung (12) es ermöglicht die alternierende Emission von Ultraschallwellen von zwei Typen zu steuern, wobei der erste, thermisch genannte Typ an dem zu behandelnden Gewebe (16) einen vorwiegend thermischen Effekt erzeugt, und der zweite vom sogenannten Kavitationstyp an den zu behandelnden Geweben eine vorwiegend aushöhlende Wirkung erzeugt.

2. Vorrichtung nach Anspruch 1, dadurch gekennzeichnet, daß die Steuereinrichtung (20, 22) die Behandlungsanordnung (12), zumindest am Anfang der Behandlung auf thermische Ultraschallwellen steuert.

3. Vorrichtung nach Anspruch 1 oder 2, dadurch gekennzeichnet, daß die Steuereinrichtung (20, 22) der Behandlungsanordnung (12) die Emission von Kavitationsultraschallwellen nach einer vorbestimmten Zeitperiode steuert, welche einstellbar ist, wobei eine Vorerwärmung der zu behandelnden Gewebe ermöglicht wird.

4. Vorrichtung nach einem der vorangegangenen Ansprüche, dadurch gekennzeichnet, daß die Steuereinrichtung (20, 22) es ermöglicht die Emission von Kavitationsultraschallwellen gleichzeitig zu der Emission von thermischen Ultraschallwellen zu steuern.

5. Vorrichtung nach einem der vorangegangenen Ansprüche, dadurch gekennzeichnet, daß die akustische Intensität der thermischen Ultraschallwellen niedriger ist als die Kavitationsschwelle, während die akustische Intensität der Kavitationsultraschallwellen mindest gleich der Kavitationsschwelle ist, wobei die Kavitationsschwelle eine Funktion der zu behandelnden Gewebe des Säugetieres ist.

6. Vorrichtung nach einem der vorangegangenen Ansprüche, dadurch gekennzeichnet, daß die Frequenz der Kavitationsultraschallwellen niedriger ist als die Frequenz der thermischen Ultraschallwellen.

7. Vorrichtung nach einem der vorangegangenen Ansprüche, dadurch gekennzeichnet, daß die Steuereinrichtung (20, 22) die Emission von Kavitationsultraschallwellen realisiert, umfassend eine negative Komponente in der Amplitude und von solch einer Art, daß sie die Kavitation auslöst.

8. Vorrichtung nach einem der vorangegangenen Ansprüche, dadurch gekennzeichnet, daß die Steuereinrichtung (20,22) die Emission von Kavitationsultraschallwellen von einer Dauer erreicht, welche enthalten ist zwischen etwa 0,5 Mikrosekunden und etwa 100 Millisekunden, und bevorzugt umfaßt zwischen 0,5 Mikrosekunden und 50 Mikrosekunden.

9. Vorrichtung nach einem der vorangegangenen Ansprüche, dadurch gekennzeichnet, daß die Steuereinrichtung (20, 22) die Emission von Kaviationsultraschallwellen erreicht durch sukzessive Impulse bei einer Wiederholungsfrequenz, welche sich verändert zwischen etwa 1 Hz und 1 kHz, bevorzugt zwischen etwa 10 Hz und 100 Hz.

10. Vorrichtung nach einem der Ansprüche 3 bis 9, dadurch gekennzeichnet, daß die Dauer des einstellbaren vorbestimmten Zeitintervalles umfaßt ist zwischen etwa 100 Millisekunden und etwa 10 Sekunden.

11. Vorrichtung nach einem der vorangegangenen Ansprüche, dadurch gekennzeichnet, daß die Gesamtbehandlungsdauer des Gewebebereiches, welcher bestimmt ist durch den Fokalpunkt oder die Fokalzone durch die Ultraschallwellen umfaßt ist zwischen 100 Millisekunden und 10 Sekunden, wobei diese Gesamtdauer zumindest einen Kaviationsultraschallwellenimpuls umfaßt.

12. Vorrichtung nach einem der Ansprüche 1 bis 11, dadurch gekennzeichnet, daß sie eine Versetzungseinrichtung (22) der Behandlungsanordnung umfaßt, so daß die Behandlung punktweise durchgeführt wird, wobei jeder Punkt bestimmt ist durch den Fokalpunkt oder die Fokalzone, so daß die Gesamtheit des Volumens des zu behandelnden Zieles abgedeckt wird.

13. Vorrichtung nach Anspruch 12, dadurch gekennzeichnet, daß die Versetzungseinrichtung (22) der Behandlungsanordnung (12) gesteuert ist durch eine Steuerzentrale (20), z.B. umfassend eine Recheneinheit wie einen Computer oder einen Mikrocomputer, wobei dieser bevorzugt über ein Programm verfügt, welches die Versetzung der Behandlungsanordnung (12) als Funktion des Volumens des zu behandelnden Zieles veranlaßt, wobei vorteilhafterweise die Volumendaten erhalten sind durch eine zugeordnete Bildeinrichtung (40), welche eine besondere Bildsteuereinrichtung (44) umfaßt, welche durch die zentrale Steuerung (20) gesteuert ist, und bevorzugt eine Versetzungseinrichtung (30), insbesondere zur Translation und/oder zur Rotation.

14. Vorrichtung nach Anspruch 13, dadurch gekennzeichnet, daß die Steuerzentrale (20) die Versetzung der Versetzungseinrichtung (22) der Behandlungsanordnung (12) derart steuert, daß die Behandlung der Gewebezonen des Zieles (16), welche am weitesten von der Behandlungsanordnung (12) entfernt sind, um die Gewebezonen zu erreichen, welche am nähesten zu der Behandlungsanordnung (12) sind, um somit die Behandlungseffizienz des Zieles zu verbessern.

15. Vorrichtung nach einem der vorangegangenen Ansprüche, dadurch gekennzeichnet, daß die Steuereinrichtung (20, 22) eine Latentzeit zwischen der Behandlung von zwei aufeinanderfolgenden Punkten des zu behandelnden Zieles sichert, so daß eine Relaxation der zu behandelnden Gewebe ermöglicht wird, wobei bevorzugt die Latentzeit umfaßt ist zwischen etwa 1 Sekunde und 15 Sekunden, wobei diese Latentzeit vorteilhafterweise verwendet wird zum Durchführen der Versetzung der Behandlungsanordnung von einem Behandlungspunkt zu einem anderen.

16. Vorrichtung nach einem der vorangegangenen Ansprüche, dadurch gekennzeichnet, daß die Zentralsteuerung (20) eine Versetzung der Versetzungseinrichtung (22) der Behandlungsanordnung (12) in einer zufälligen Weise steuert, wobei die bereits behandelnden Punkte ausgeschlossen sind.

17. Vorrichtung nach einem der vorangegangenen Ansprüche, dadurch gekennzeichnet, daß die Emissionsfrequenz der Kavitationsultraschallwellen umfaßt ist zwischen etwa 500 kHz und 4 MHz, bevorzugt zwischen 500 kHz und 2 MHz, und insbesondere bevorzugt von etwa 1 MHz.

18. Vorrichtung nach einem der vorangegangenen Ansprüche, dadurch gekennzeichnet, daß die Emissionsfrequenz der thermischen Ultraschallwellen umfaßt ist zwischen etwa 1 und 4 MHz, wobei diese Frequenz zumindest gleich der Frequenz der Kavitationsultraschallwellen ist.

19. Vorrichtung nach einem der vorangegangenen Ansprüche, dadurch gekennzeichnet, daß die akustische Intensität der thermischen Ultraschallwellen geringer ist als etwa 150 W/cm², und daß die akustische Intensität der Kavitationsultraschallwellen zumindest gleich etwa 150 W/cm² ist.

20. Vorrichtung nach einem der vorangegangenen Ansprüche, dadurch gekennzeichnet, daß die Steuereinrichtung (20, 22) die Emission von Ultraschallwellen mit variabler Amplitude erreicht, und zwar als Funktion der Zeit, bevorzugt mit anwachsender Amplitude im Laufe der Zeit, so daß die Amplitude während einer ersten Periode (T₁, Fig. 6) geringer als die Kavitationsschwelle (SC_{T}) bleibt, wonach in einer zweiten Periode (T₂, Fig. 6) sie größer als die Kavitationsschwelle (SC_{T}) wird.

21. Ultraschalltherapievorrichtung (110) nach einem der Ansprüche 1 bis 20, umfassend zumindest eine Behandlungsanordnung (116), welche vorgesehen ist zum Durchführen zumindest der Therapie angesichts der Zerstörung eines zu zerstörenden Zieles (112), sowie die Gewebe (P), welche sich im Inneren des Körpers eines Säugetieres (M) befinden können, insbesondere in einem menschlichen Wesen, und eine Steuereinrichtung (120, 122) der Anordnung (116) zum Durchführen der Therapie, wobei das piezoelektrische Transducerelement (118) vorgesehen ist zum Liefern von fokussierten Ultraschalltherapiewellen von hoher akustischer Energie bei einem Fokalpunkt oder eine Fokalzone (F), welche die Gewebezone (P) bestimmt, welche mit der Therapie zu behandeln ist, wobei die Ultraschallwellen Gewebezonen (114) durchqueren, welche an der Schnittstelle zu der Therapieanordnung angeordnet sind, dadurch gekennzeichnet, daß sie umfaßt eine Abkühleinrichtung (130), vorteilhafterweise mit einem Kühlfluid (132), bevorzugt ein flüssiges Kühlmittel, wie Wasser, welches erlaubt die Abkühlung in einem vorbestimmten Temperaturbereich zu erreichen, und zwar zumindest der Gewebezonen (114), welche sich an der Schnittstelle zu der Therapieanordnung (116) befinden, wodurch in effizienter Weise es ermöglicht wird, die Gewebezonen zu schützen, welche an der Schnittstelle angeordnet sind, und zwar gegen die Kavitationswirkungen.

22. Vorrichtung nach einem der Ansprüche 1 bis 21, dadurch gekennzeichnet, daß die Therapieanordnung (12, 116) extrakorporell bzw. äußerlich anwendbar ist.

23. Vorrichtung nach einem der Ansprüche 1 bis 21, dadurch gekennzeichnet, daß die Therapieanordnung (116) eine endokavitive Anordnung ist, welche eine Therapie über halbinvasivem Wege erlaubt, wobei die endokavitive Anordnung insbesondere eine endorektale oder endouretrale oder ösophagische Anordnung ist.

24. Vorrichtung nach einem der vorangegangenen Ansprüche, dadurch gekennzeichnet, daß sie zumindest eine endokavitive Anordnung (160) umfaßt, welche physikalisch unabhängig von der Therapieanordnung (12; 116) ist, um die Gewebezonen (U) abzukühlen, welche entfernt von der Therapieanordnung sind, und welche man ebenfalls während der Therapie schützen möchte.

25. Vorrichtung nach einem der Ansprüche 1 bis 24, dadurch gekennzeichnet, daß sie umfaßt zumindest eine Temperaturmeßeinrichtung (148) für Gewebe (114), welche an der Schnittstelle mit der Therapieanordnung (12; 116) angeordnet sind, eine Empfangs-Übertragungseinrichtung (142) für Temperaturdaten, welche übertragen werden durch die Temperaturmeßeinrichtung, und zwar zu einer Steuerzentrale (120), welche in der Lage ist die Befehlsinstruktionen bzw. Steuerbefehle der Betätigung der Therapieanordnung als Funktion der empfangenen Temperaturdaten zu modifizieren.

26. Vorrichtung nach Anspruch 25, dadurch gekennzeichnet, daß die Temperaturmeßeinrichtung (148) Sensoren umfaßt, die in einer Form von Thermopaaren oder in der Form eines Blattes, insbesondere des Types PVDF, welche direkt an den Gewebezonen der Schnittstelle mit Bezug auf die Therapieanordnung angeordnet sein können, oder an der äußeren Fläche der Membran (134), welche die Abkühl- bzw. Kühlflüssigkeit (132) umschließt, und welche an den Geweben der Schnittstelle (114) anliegt, bevorzugt zumindest eine Drucksensoreinrichtung, insbesondere des Types PVDF, welche es erlaubt den akustischen Druck des Ultraschallfeldes zu messen, welches emittiert ist durch die Therapieanordnung (116) auf der Höhe der Schnittstelle (114), so daß die elektrische Leistung geregelt ist, welche an das Transducerelement (118) geliefert ist zum Aufrechterhalten eines konstanten Wertes des Druckes des akustischen Ultraschallfeldes am Fokalpunkt bzw. Brennpunkt (F).

27. Vorrichtung nach einem der vorangegangenen Ansprüche, dadurch gekennzeichnet, daß sie verwendet oder angewendet wird auf sämtliche Ultraschalltherapien, welche bevorzugt lokalisiert sind, und zwar von sämtlichen gutartigen oder bösartigen Tumoren, externen oder internen, insbesondere bevorzugt für die Behandlung von gutartigen oder bösartigen Tumoren der Leber, der Prostata, der Niere, der Brust, der Haut, des Gehirnes und die Behandlung von Krampfadern sowie des Ösophages bzw. der Speiseröhre.

28. Verfahren zum Steuern einer Ultraschalltherapievorrichtung, welche aufweist zumindest ein piezoelektrisches Transducerelement (14) und eine Steuereinrichtung des oder der Transducerelemente (14) zum Steuern der Emission über den oder die Transducer von Therapieultraschallwellen hin zu behandelnden Geweben, dadurch gekennzeichnet, daß das Verfahren die folgenden aufeinanderfolgenden oder gleichzeitigen Schritte umfaßt:
- Steuern der Emission über den oder die Transducer von Ultraschalltherapiewellen, welche maßgeblich eine thermische Wirkung in den zu behandelnden Geweben aufweist;
- Steuern der Emission über den oder die Therapieultraschallwellentransducer, welche maßgeblich eine Kavitationswirkung in den zu behandelnden Geweben aufweist.
